# EUROPÄISCHE PATENTSCHRIFT

(11) **EP 0 617 931 B1**
(45) Veröffentlichungstag und Bekanntmachung des Hinweises auf die Patenterteilung: **13.01.1999**
(21) Anmeldenummer: 94105026.2
(22) Anmeldetag: 30.03.1994
(51) Int. Cl.: A61F 2/34

(54) **Künstliche Hüftgelenkspfanne**
Artificial hip acetabular cup
Coque acétabulaire artificielle de hanche

(30) Priorität: 31.03.1993 DE 4310592
(43) Veröffentlichungstag der Anmeldung: 05.10.1994
(73) Patentinhaber: Wessinghage, Dieter, Prof. Dr. med., D-93051 Regensburg (DE)
(72) Erfinder: Wessinghage, Dieter, Prof. Dr. med., D-93051 Regensburg (DE)
(74) Vertreter: Meier, Gerald Heinz

(56) Entgegenhaltungen:
- CH-A- 649 913
- DE-A- 3 714 468
- DE-A- 3 821 720
- FR-A- 2 134 170
- FR-A- 2 637 177
- FR-A- 2 641 461
- GB-A- 2 080 118

## Beschreibung

Die Erfindung betrifft eine künstliche Hüftgelenkspfanne zur Verankerung im Acetabulum des menschlichen Beckens, wobei die Verankerung mittels eines aushärtbaren Knochenzementes oder mittels den äußeren Teil der Hüftgelenkspfanne durchdringender Schrauben erfolgt.
Eine gattungsgemäße künstliche Hüftgelenkspfanne, entsprechend dem Oberbegriff des Patentanspruches, ist aus der GB-A-2080118 bekannt.

Die bekannten künstlichen Hüftgelenkspfannen für den angegebenen Verwendungszweck lassen sich prinzipiell nach der Art der Verankerung im Becken in zementierbare und verschraubbare Hüftgelenkspfannen unterteilen.

Zementierbare Hüftgelenkspfannen sind aus der FR-A-2637177 sowie der oben erwähnten GB-A-2080118 bekannt. Sie weisen in ihrer Außenfläche zur Rotationsachse konzentrisch umlaufende bzw. teilweise umlaufende Nuten auf. Beim Einsetzen der Hüftgelenkspfanne in ein Zementbett im Acetabulum des Beckenknochens soll Zement in diese Nuten eindringen und dadurch eine sichere und vor allem dauerhafte Befestigung der Pfanne erreicht werden. Die sogenannte "Überlebenszeit" der Prothese, das größte Problem bei der Implantation von Endoprothesen, ist jedoch nicht nur von einem optimalen Kunstpfannen-Zement-Verbund, sondern mindestens ebenso von einem optimalen Knochen-Lager-Zement-Verbund abhängig.
Es hat sich jedoch in der operativen Praxis gezeigt, daß beim Einsetzen solcher Pfannen der eine relativ zähe Konsistenz aufweisende Zement zumindest teilweise an den Nuten der Kunststoffpfannen vorbeiläuft und partiell nichtausgefüllte Hohlräume bleiben, die die Festigkeit des Verbundes beeinträchtigen.
Bei ungenügendem Knochen-Zement-Implantat-Verbund können sich auch im weiteren Verlauf sogenannte röntgenologische Aufhellungsspalten ausbilden. Diese Aufhellungssäume können unter Bewegung und Belastung über lokale Mikrobewegungen zu größerer Instabilität führen.
Insgesamt ist es bei den bekannten zementierbaren Hüftgelenkspfannen nicht oder nicht vollständig möglich, die Hohlräume, d.h. die Nuten, vollständig mit Knochenzement zu füllen.

Weiterhin sind aus der Firmenschrift "METASUL", Ausgabe 1/1992, der Fa. PROTEK auch verschraubbare Hüftgelenkspfannen bekannt. Diese weisen Schrauben auf, die den äußeren Teil der Hüftgelenkspfanne durchdringen und in den Knochen des Acetabulums gebohrt werden. Die dem Becken zugewandte Außenfläche solcher verschraubbarer Hüftgelenkspfannen ist dabei glatt oder auch konturiert ausgeführt.
Es können auch Knochenspäne oder Knochenersatzstoffe in die Ausfräsung des Beckenknochens eingebracht werden, die - analog dem Knochenzement bei zementierbaren Hüftgelenkspfannen - die verbleibenden Hohnräume möglichst vollständig ausfüllen sollen. Dies ist bei den bekannten Metallpfannen nicht oder nicht vollständig möglich.

Aufgabe der Erfindung ist es demnach, eine Hüftgelenkspfanne der gattungsgemäßen Art anzugeben, die einfach herstellbar und leicht in das zerstörte bzw. ausgefräste natürliche Acetabulum einsetzbar ist und dabei mit Sicherheit beim Einsetzen bzw. Befestigen und dem daraus resultierenden Bewegen der Außenseite der Pfanne hin zum Beckenknochen das vollständige Ausfüllen des Zwischenraumes mit fließfähiger und partiell komprimierbarer Substanz, nämlich Knochenzement oder -material, gestattet.

Diese Aufgabe wird erfindungsgemäß durch eine Hüftgelenkspfanne mit den Merkmalen des Patentanspruches gelöst.

Der Hauptvorteil der Erfindung liegt insbesondere darin, daß der Knochenzement bzw. das Knochenmaterial- im Unterschied zu den bereits bekannten Pfannen - beim Einsetzen so zwangsgeführt und gleichzeitig verdichtet wird, daß ein vollständiges Ausfüllen der Nuten sicher gewährleistet ist.
Erfindungsgemäß sind zusätzlich zu den per se bekannten konzentrischen Nuten Quernuten vorgesehen, die meridianartig, d.h. auf Großkreisen, durch den Pol der Hüftgelenkspfanne verlaufen und einen Querschnitt aufweisen, der sich polabwärts kontinuierlich verringert. Dadurch wird beim Einsetzen der Pfanne eine Zwangsführung bewirkt; der Zement wird durch den entgegen der Eindrückrichtung immer geringer werdenden Querschnitt der Quernuten beim Eindrücken verdichtet und in die beidseitigen Nutenabschnitte verpreßt.
Gleichzeitig wird durch den sich verjüngenden Querschnitt der Zement zusätzlich zur Auffüllung der Knochenhohlräume in die Tiefe gepreßt.
Die Querschnitte selbst können dabei beispielsweise dreieckig oder auch trapezförmig sein; die erfindungsgemäße Verringerung des Querschnittes mit wachsendem Abstand vom Pol kann sowohl durch eine Verringerung der Tiefe der Quernut als auch ein Zusammenlaufen der Seitenbegrenzungen erfolgen.

Die Erfindung soll nachstehend anhand von Zeichnungen beispielhaft näher erläutert werden.

Die Figuren zeigen:
- Fig. 1: eine erfindungsgemäße künstliche Hüftgelenkspfanne in seitlicher Darstellung, wobei die Einzelheit A aus Figur 1 gesondert in Fig. 1a dargestellt ist.
- Fig. 2: die Einzelheiten B und C der Fig. 1 in einer ersten Ausführungsform jeweils in Schnittdarstellung senkrecht zur Oberfläche
- Fig. 3: die Einzelheiten B und C in einer zweiten Ausführungsform in ebensolcher Darstellung.

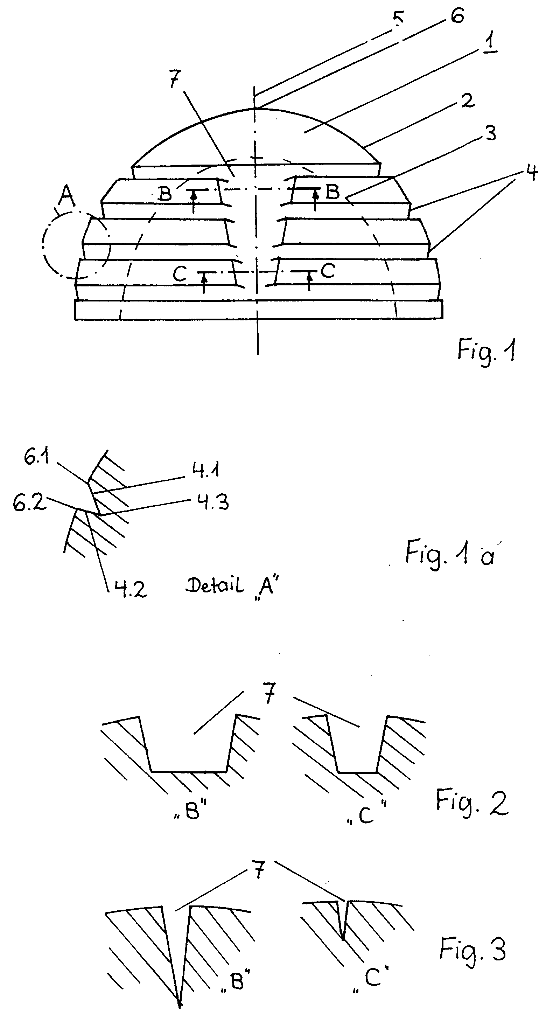

Die in Fig. 1 dargestellte Hüftgelenkspfanne 1 weist eine in etwa halbkugelförmige Außenfläche 2 und eine, nur andeutungsweise dargestellte, Innenflache 3 auf, die einen - nicht dargestellten -Femurkopf aufnimmt
Die Hüftgelenkspfanne 1 besteht üblicherweise aus Polyäthylen oder einem anderen Kunststoff. In der Außenfläche 2 befinden sich konzentrische Nuten 4 mit einem erfindungsgemäßen Querschnitt. Die beiden Flanken 4.1 und 4.2 jeder sich ins Innere der Hüftgelenkspfanne 1 erstreckenden Nut 4 verjüngen sich in ihrem Abstand zueinander von außen nach innen. Dabei weisen die Flanken 4.1, 4.2 von der Vertikalen bzw. von der Horizontalen abweichende Neigungswinkel auf. Die Nuten 4 laufen im Inneren spitz zu, dabei ist die Spitze zur polabgewandten Seite gerichtet.

Die jeweiligen Begrenzungsränder jeder Nut 4 sind besonders vorteilhaft als obere und untere Schneidkanten 6.1, 6.2 ausgebildet.
Die Flanken 4.1, 4.2 jeder Nut 4 laufen im Inneren zu einem spitzwinkligen Scheitel 4.3 zusammen.
Die zwischen den jeweiligen Nuten 4 liegenden Segmente der Außenfläche 2 weisen eine kugelige Oberfläche auf. In der Außenfläche 2 sind dabei mehrere Quernuten 7, sich meridianartig vom Pol 6 zum offenen Ende erstreckend, vorgesehen. Vom Pol 6 weg verringert sich deren Querschnitt kontinuierlich, so daß beim Einpressen der Hüftgelenkspfanne 1 der Knochenzement in den Quernuten 7 immer mehr verdichtet und zwangsweise in die konzentrischen Nuten 4 verpreßt wird. Durch die erfindungsgemäße Kombination der konzentrischen Nuten 4 mit den Quernuten 7 ist eine ideale Kompression des Knochenzementes beim Einsetzen und eine vollständige, hohlraumfreie Verfüllung aller Nuten 4 und Quernuten 7 gewährleistet.

## Patentansprüche

1. Künstliche Hüftgelenkspfanne (1) zur Verankerung im Acetabulum des menschlichen Beckens, wobei die Verankerung mittels eines aushärtbaren Knochenzementes oder mittels die Außenfläche (2) der Hüftgelenkspfanne (1) durchdringender Schrauben erfolgt,
mit zumindest teilweise umlaufenden konzentrischen Nuten (4) an der Außenfläche (2), wobei diese Nuten (4) im Querschnitt durch jeweils zwei zumindest annähernd ebene Flanken (4,1, 4,2) gebildet sind,
wobei sich die Nuten (4) von der Außenfläche (2) weg ins Innere erstrecken,
wobei zwischen den konzentrischen Nuten (4) ebenfalls konzentrische Bereiche der kugelförmigen Außenfläche (2) stehenbleiben,
wobei zumindest jeweils eine der Flanken (4.1, 4.2) einen von der Horizontalen bzw. Vertikalen abweichenden Neigungswinkel aufweist, so daß sich der Querschnitt der Nuten (4), d.h. der Abstand der Flanken (4.1, 4.2) zueinander, kontinuierlich verringert,
und wobei die Flanken (4.1, 4.2) im Inneren der Hüftgelenkspfanne (1) spitzwinklig bis zu einem ebenfalls spitzwinkligen Scheitel (4.3) zusammenlaufen,
***dadurch gekennzeichnet,***
daß der spitzwinklige Scheitel (4.3) zur polabgewandten Seite der Hüftgelenkspfanne (1) gerichtet ist
und daß zusätzliche Quernuten (7) vorgesehen sind, die sich meridianartig vom Pol (6) weg mit einem vom Pol (6) weg sich kontinuierlich verringernden Querschnitt erstrecken.

## Claims

1. Artificial hip acetabular cup (1) for anchoring in the acetabulum of the human pelvic girdle, wherein the anchoring is effected by means of a hardenable bone cement or by means of screws passing through the outer surface (2) of the hip acetabular cup (1), with at least partially encircling concentric grooves (4) at the outer surface (2), wherein these grooves (4) are each formed in cross-section by two at least approximately planar flanks (4.1, 4.2), wherein the grooves (4) extend away from the outer surface (2) and into the interior, wherein similarly concentric regions of the spherical outer surface (2) are left between the concentric grooves (4), wherein each time at least one of the flanks (4.1, 4.2) has an angle of inclination deviating from the horizontal or the vertical, so that the cross-section of the grooves (4), i.e. the spacing of the flanks (4.1, 4.2) from one another, continuously reduces, and wherein the flanks (4.1, 4.2) converge in the interior of the hip acetabular cup (1) at an acute angle up to a similarly acute-angled apex (4.3), characterised in that the acute-angled apex (4.3) is directed towards the side of the hip acetabular cup (1) remote from the pole and that additional transverse grooves (7) are provided, which extend in the manner of meridians away from the pole (6) with a cross-section continuously reducing away from the pole (6).

## Revendications

1. Cotyle artificielle de hanche (1) destiné à être ancré dans l'acétabule du bassin d'un être humain, l'ancrage ayant lieu au moyen d'un ciment osseux durcissable ou au moyen de vis passant à travers la surface extérieure (2) du cotyle de la hanche (1), avec des rainures (4) concentriques qui font au moins en partie le tour sur la face extérieure (2), ces rainures (4) étant formées en section transversale par respectivement deux flancs (4.1,4.2) plats, au moins approximativement,
- les rainures (4) s'étendant à l'intérieur au moins à partir de la surface extérieure (2),
- des zones également concentriques des surfaces extérieures (2) en forme de sphère restant immobiles entre les rainures concentriques (4),
- au moins l'un des flancs (4.1,4.2) présentant respectivement un angle d'inclinaison qui s'écarte de l'horizontale ou de la verticale, de telle sorte que la section transversale des rainures (4), c'est à dire l'écart entre les flancs (4.1,4.2), l'un par rapport à l'autre, se rétrécit de façon continue et
- les flancs (4.1,4.2) convergeant à l'intérieur du cotyle de la hanche (1) en formant un angle aigu jusqu'à un sommet (4.3), formant également un angle aigu,
caractérisée en ce que
- le sommet (4.3), qui forme un angle aigu, est orienté en direction du côté du cotyle de la hanche (1) qui est situé à l'opposé de son pôle et
- des rainures transversales additionnelles (7) sont prévues, rainures qui s'étendent à la manière de méridiens à partir du pôle (6) avec une section transversale qui va en se rétrécissant de façon continue en s'éloignant du pôle (6).
